# EUROPEAN PATENT APPLICATION

(11) **EP 2 446 735 A2**
(43) Date of publication of application: **02.05.2012**
(21) Application number: 10792250.2
(22) Date of filing: 12.03.2010
(51) Int. Cl.: A01H 1/06, A01H 5/00

(54) **NOVEL PLANT VARIETY OF HIGH FERTILITY XBRASSICORAPHANUS WITH STABILIZED SEED PRODUCTIVITY**

(30) Priority: 23.06.2009 KR 20090055969
(71) Applicant: Biobreeding Co., Ltd., Gyeonggi-do 456-756 (KR)
(72) Inventor: LEE, Soo-Seong, Seongnam-si Gyeonggi-do 463-030 (KR)
(74) Representative: Bentham, Andrew
(86) International application number: PCT/KR2010/001568
(87) International publication number: WO 2010/150968

(57) **Abstract**

The present invention relates to a method for breeding a novel plant variety of high fertility *xBrassicoraphanus* with stabilized seed productivity, and discloses a method for breeding a novel plant variety of *xBrassicoraphanus,* which involves performing a mutagen treatment on *xBrassicoraphanus* seeds by treating the *xBrassicoraphanus* seeds with 0.01 µg/L of mutagen NMU, sowing the *xBrassicoraphanus* seeds, gathering the *xBrassicoraphanus* seeds, sowing the *xBrassicoraphanus* seeds again, performing artificial crossing in an indoor area to check seed productivity, and checking the uniformity of the variety using an AFLP primer.

## Description

### [RELATED APPLICATIONS]

This application claims priority from Korean Patent Application No. 10-2009-0044969 filed on June 23, 2009 in the Korean Intellectual Property Office, the entire disclosure of which is incorporated herein by reference.

### [BACKGROUND OF THE INVENTION]

### 1. Field of the Invention

The present invention relates to a novel plant variety of *xBrassicoraphanus* with stabilized seed productivity.

### 2. Description of the Related Art

The present inventors have cultivated *'xBrassicoraphanus* (genus, genome: aarr, 2n=38)', which is an intergeneric hybrid plant between *Brassica campestris* and *Raphanus sativus,* and acquired a patent right for the same (Korean Patent No. 0492518, entitled "Novel modified variety of *xBrassicoraphanus* and a method for breeding the same," patented on May 23, 2005). Thereafter, it was confirmed by the inventors that the foregoing plant may be widely used for sprout vegetables, baby vegetables, wrapping vegetables, young radish vegetables, vegetables for preparation of kimchi, or as a raw material for health supplements. However, common two problems involved in remote-crossed hybrids were not completely overcome.

Among such problems, one is low seed productivity. In fact, the proposed plant, that is, *xBrassicoraphanus* produces plenty of seeds to make a profit in an aspect of agricultural business (400 to 600 L/ha). However, although about 10 seeds per pod may be generated, only 1 to 3 seeds are ripe while the others atrophy (or are degraded), thus reducing the quantity of seeds to be obtained. The other problem is to be that traits are continuously segregated, thus decreasing uniformity. Although the uniformity is to be a level with no difficulty in agricultural utilization, features of the seed are gradually and continuously segregated through successive generations thereof. From this, there was found a problem in that different traits must thin out during seed gathering and cultivation.

### [SUMMARY OF THE INVENTION]

Therefore, in order to overcome the foregoing problems of the related art, the present invention is directed to breeding and providing a novel plant lineage ('line') of which all seeds in a pod are ripe without degradation thereof and which ensures uniformity sufficient to use the seeds as a parent of first filial generation (F1) hybrid variety according to an appropriate breeding method, like general plants, in view of botanical aspect.

In order to accomplish the above object, the present invention provides a breeding method including: mutagen treatment (that is, mutation); gathering seeds; artificial crossing to check seed productivity; and checking the uniformity of the variety using an AFLP primer.

### [BRIEF DESCRIPTION OF THE DRAWINGS]

The above and other objects, features and other advantages of the present invention will be more clearly understood from the following detailed description taken in conjunction with the accompanying drawings, in which:
FIG. 1 is a photograph showing a condition of seeds in hydrothecae in each line after gathering seeds of *xBrassicoraphanus* in field; and
FIG. 2 is photographs showing cultivation of a novel plant variety of *xBrassicoraphanus* to assay horticultural characteristics thereof.

### [DETAILED DESCRIPTION OF THE INVENTION]

### Experimental material and procedure

Microspore mutation was adopted as a breeding method of a novel plant variety of *xBrassicoraphanus.* For microspore culture developed by the present inventors, a rate of blastogenesis was only 0.9 blastocyst per Petri dish (Hong and Lee, 1995). Thus, the foregoing method is unsuitable for inducing mutation. Accordingly, an improved culturing method to produce embryos in larger quantities was established and an experiment to treat the produced embryos with of 0.01 µg/L of a mutagen MNU was executed. Among numerous experiments, a particular one to produce a large quantity of embryos was used and the produced embryos were induced into a plant and a successive one thereof was subjected to seed gathering. As a result, compared to existing *xBrassicoraphanus,* individuals with a large seed gathering amount were observed in artificial crossing in an indoor area (see, Tables 15-23, pp. 31-38; Agricultural Research and Development Promotion Center (ARPC) subject, "Improvement of major traits of a novel vegetable, *xBrassicoraphanus* and all year round production thereof as a wrapping vegetable," final report (2006)).

Under the inspiration of the foregoing experimental results, a number of intensive and extensive studies have been conducted since 2007. As a result, for four (4) individuals showing a large seed gathering amount in 2007, successive ones were subjected to artificial crossing in an indoor area to identify seed production abilities thereof. In an autumn season, they were cultivated in a package to assay uniformity thereof. Next, the foregoing lines were cultivated in fields in 2008, to identify seed production abilities in natural environments. Since it was deemed that visual inspection of the uniformity of each of the lines only may be insufficient, comparison assay was performed through amplified fragment length polymorphism (AFLP) using nine (9) primers such as - AAC/M-CAA to CGG.

### Identification and assay of uniformity and seed productivity

In an artificial crossing test in an indoor area to identify seed productivity, seeds were harvested in large quantities from each line treated using a mutagen, which were substantially similar to the previous year (TABLE 1).

**TABLE 1**

| Systematic name | Systematic classification | Number of hybridization | Number of seeds | Number of seeds per hybridization | Note |
|---|---|---|---|---|---|
| BB #1 | Preserved line | 274 | 106 | 0.39 | Artificial anthesis pollinat ion |
| BB #11 | Mutation line | 274 | 881 | 3.2 | |
| BB #12 | " | 379 | 1118 | 3.0 | |
| BB #13 | " | 227 | 781 | 3.4 | |
| BB #14 | " | 231 | 1009 | 4.37 | |
| BB #50 | " | 229 | 786 | 3.43 | |

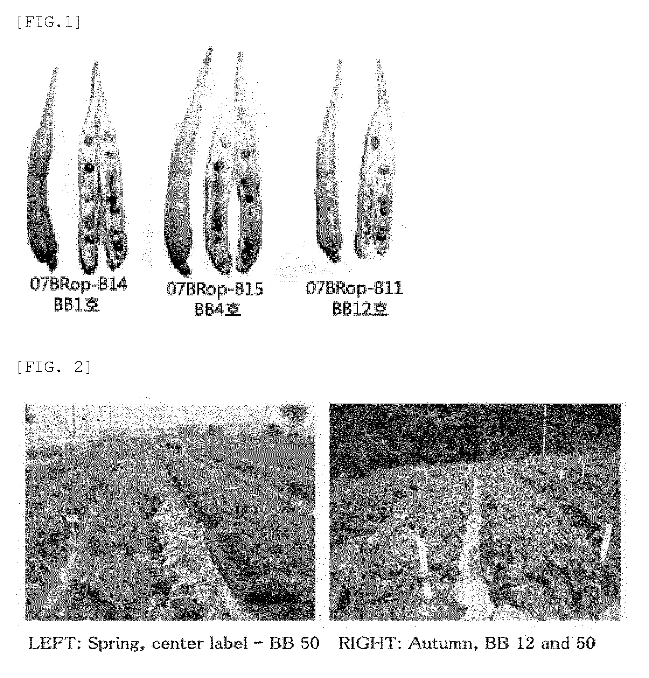

As a result of cultivating the foregoing lines in autumn, they exhibited remarkably improved uniformity, compared to existing lines. Therefore, in the next year, four new lines as well as two existing lines were cultivated in the same field and compared to each other in respects to seed production ability under the same conditions and in natural environments (see TABLES 2 and FIG. 1).

**TABLE 2 - Comparison of seed gathering ability in field between different xBrassicoraphanus lines**

| system | Systematic classification | Number of commercial individuals | Number of seeds | | | |
|---|---|---|---|---|---|---|
| | | | Total amount (mL) | Per hydrothecae (ea) | Per plant (mL) | 10a (L) |
| BB #4 | Preserved line | 21 | 630 | 1.6 | 30.0 | 60.0 |
| BB #1 | " | 39 | 1.140 | 2.3 | 29.2 | 58.5 |
| BB #11 | Mutation line | 30 | 1.790 | 7.6 | 59.7 | 119.3 |
| BB #12 | " | 40 | 3.280 | 8.8 | 82.0 | 164.0 |
| BB #13 | " | " | 2.810 | 7.6 | 70.3 | 140.4 |
| BB #14 | " | " | 2.140 | 8.0 | 53.5 | 107.0 |

From the foregoing, a production amount of each of existing lines BB 1 and 4 was about 60L in respects to a total production amount of 10a, and substantially similar to test results in the art. On the other hand, each of four lines treated using mutagens, that is, BB 11, 12, 13 and 14, respectively, exhibited a production amount of 100L or more. Specifically, BB 12 produced about 160L or more, which is substantially similar to an amount of fixed species of *Raphanus sativus* or *Brassica campestris.* The reason for such results is found from FIG. 1 to be because seeds in the pods were almost not degraded but ripe. For BB 1 and 4, most seeds were degraded in brown, while BB 12 showed very little degradation of seeds and most of the seeds were still green. As such, even it was a remote-crossed hybrid, seed productivity was improved to a level completely identical to existing plants. That is, one of the problems of the remote-crossed hybrid was successfully solved.

Although the second problem (of the remote-crossed hybrid), that is, low uniformity is also concerned, it was found that the uniformity was noticeably increased in package cultivation executed twice, compared to existing lines. More particularly, in outward appearance, the foregoing exhibited substantially similar uniformity to F1 hybrid of *Raphanus sativus* or *Brassica campestris.* In order to obviously demonstrate such results, AFLP technique was applied. In the case where all commercial individuals of one line are treated through electrophoresis by AFLP, these will exhibit the same band if they have the same genes. On the other hand, if there is an individual having even one different gene, the individual may show a different band from those of other individuals, that is, a polymorphic band. AFLP was executed on the basis of the foregoing theory and, as expected, BB 4 deemed to have the lowest uniformity was identified to have a polymorphic band rate of 5.4%, which is the highest level among them.

**TABLE 3 - Verification of purity of xBrassicorphanus line by AFLP**

| Systematic name | Number of individuals for verification | AFLP bands | | Polymorphic band rate (%) |
|---|---|---|---|---|
| | | Total | Polymorphism between individuals | |
| BB #4 | 21 | 919 | 50 | 5.4 |
| BB #1 | 39 | 672 | 30 | 4.5 |
| BB #11 | 30 | 731 | 31 | 4.2 |
| BB #12 | 40 | 673 | 33 | 4.9 |
| BB #13 | 40 | 673 | 25 | 3.7 |
| BB #14 | 40 | 729 | 29 | 4.0 |

However, BB 1 also exhibited the polymorphic band rate of 4.5%, although the above line was considered to have the highest uniformity among existing preserved lines. Further, lines having remarkable improvement in seed productivity among those obtained after mutagen treatment exhibited the seed productivity ranging from 4.0% to 4.9%, which is substantially less than 5.0%. Studies upon use of AFLP techniques as a reference indicating uniformity of (plant) varieties have been executed. Although F1 hybrid with highest uniformity and both parents thereof have different polymorphic band rates along with materials and test equipment, it is known that they mostly show a seed productivity up to 5%. Accordingly, it can be seen that BB 11, 12, 13 and 14 as well as BB 1 may have improved uniformity, which is substantially identical to outward appearance thereof (see FIG. 2). Consequently, the second problem, that is, low uniformity was completely overcome.

With regard to four (4) lines presumed to be mutated by a mutagen, there may be a question how all traits are fixed in a first modified generation (M1) and become uniform. The foregoing is resulted from experimentations based on a report of Swanson et al. (1989) disclosing that fixed lines having herbicides tolerance have been bred using a mutagen in microspore culture of a rape. That is, the reason for the foregoing question is to be that microspores as haploidic cells and/or microspore-derived haploids show mutation occurred in a division period by mixing a mutagen in a culture medium for incubating microspores and using the mixed medium, which in turn grows into embryos and becomes diploids while being differentiated into a plant.

As described above, the present invention attains excellent effects in that a remote-crossed hybrid plant of *Brassica campestris* and *Raphanus sativus,* that is, *xBrassicoraphanus* exhibits stabilized seed productivity by adopting microspore mutation as a breeding method, thus producing a novel and improved line with high fertility. Moreover, the present invention exhibits additional excellent effects in that alternative novel and modified plant lines of *xBrassicoraphanus* may be bred according to the same breeding method. Therefore, the present invention may be remarkably useful for horticultural plant breeding industrial applications.

As set forth above, the present invention attains beneficial effects such as improvement in seed productivity since all seeds in each pod of *xBrassicoraphanus* are completely ripe without degradation thereof, as well as maintaining uniformity without segregation of traits.

## Claims

1. A method for breeding a novel plant variety of high fertility *xBrassicoraphanus* with stabilized seed productivity, comprising:
performing a mutagen treatment on *xBrassicoraphanus* seeds by treating the *xBrassicoraphanus* seeds with 0.01 µg/L of mutagen NMU;
sowing the *xBrassicoraphanus* seeds and gathering the *xBrassicoraphanus* seeds;
performing artificial crossing in an indoor area to check seed productivity; and
checking the uniformity of the variety using an AFLP primer.
